# EUROPEAN PATENT SPECIFICATION

(11) **EP 1 750 668 B1**
(45) Date of publication and mention of the grant of the patent: **17.02.2010**
(21) Application number: 05731023.7
(22) Date of filing: 13.04.2005
(51) Int. Cl.: A61K 9/10, A61K 47/08

(54) **LIQUIDS CONTAINING SUSPENDED GLASS PARTICLES**
FLÜSSIGKEITEN MIT SUSPENDIERTEN GLASTEILCHEN
LIQUIDES CONTENANT DES PARTICULES DE VERRE EN SUSPENSION

(30) Priority: 13.04.2004 GB 0408199; 07.03.2005 GB 0504501
(43) Date of publication of application: 14.02.2007
(73) Proprietor: Nova Bio-Pharma Technologies Limited, Lynch Wood Peterborough PE2 6PZ (GB)
(72) Inventor: ROSER, Bruce Joseph, Cambridge, Cambridgeshire CB3 9LW (GB)
(74) Representative: Lamb, Richard Andrew
(86) International application number: PCT/GB2005/050050
(87) International publication number: WO 2005/099669

(56) References cited:
- WO-A-01/37804
- US-A1- 2002 188 281
- US-B1- 6 190 701

## Description

This invention relates to a formulation comprising an active ingredient preserved in particles of a glassy or amorphous substances suspended in a liquid.

It is well known that sugar glass has an ability to preserve certain organic, biological, botanical and protein materials and there is a considerable amount of literature devoted to theoretical proposals for using this property of sugar glass to reserve pharmaceutical products, particularly vaccines. Other glassy substances have been shown to have a similar preservative effect.

Because the most commonly accepted method of administering vaccines is by injection it has been proposed, eg in patent specification WO 02/32402 (Roser) to suspend particles of water soluble glass, containing the vaccine, in a liquid (a perfluorocarbon such as perfluorodecalin) so as to create an injectable formulation. Perfluorocarbons were proposed because they are very stable and known as being safe for pharmaceutical and medical uses. It was also proposed in patent specification PCT WO 02/32402 to increase the density of the glass by adding calcium phosphate (density about 2.7 to 2.8) to the sugar glass (density about 1.5) so as to produce particles matched to the 1.97 density value of the liquid in which they were to be suspended; thereby keeping them in suspension.

A further example can be found in WO 01/37804, which discloses a formulation for storage and delivery of bioactive material, where the formulation is preserved in amorphous glass foam or particles and is suspended in a non-aqueous, hydrophobic, non-toxic liquid. This liquid is preferably fluorodecalin or cremophor EL. US 2002/188281 discloses bioactive agents in a form for administration to at least a portion of the pulmonary air passages of a patient which includes the stabilisation of the bioactive agents in amorphous glass particles which are suspended in a fluorocarbon liquid vehicle, which is preferably not a fluorinated ether but perfluorooctylbromide, dichlorofluorooctane or perfluorooctyl ethane.

The above techniques, show great promise, but the complete stability of perfluorocarbons mean that they are persistent in the troposphere and, if used in large amounts could actually contribute to global warming. In addition hydrophilic glass microsphere particles show a slight tendency to aggregate in perfluorocarbons, which are intensely hydrophobic.

According to this invention there is provided a formulation comprising an active ingredient preserved in glassy or amorphous particles, the particles being suspended in a liquid in which at least one component comprises a fluorinated ether.

Preferably, the fluorinated ethers hydrofluoroethers or hydrofluoropolyethers are considered ideal and accordingly there is provided a formulation comprising an active ingredient preserved in glassy or amorphous particles, the particles being suspended in a liquid comprising a hydrofluoroether or hydrofluoropolyether.

The inventors discovered that when mixed glass particles were added to a hydrofluoroether or hydrofluoropolyether, they dispersed astonishingly easily to form a milky suspension with little or no signs of clumping of the glass particles even after the suspension had been left for some time.

The inventors have now developed the theory that the glass particles have a hydrophilic surface whilst the perfluorocarbons, previously used, are intensely hydrophobic. For this reason, in the earlier experiments with perfluorocarbons, it is now believed that the glass particles had a tendency to clump together because they are repelled by the hydrophobic nature of the perfluorocarbon. Fluorinated ethers, behave somewhat more like a detergent, facilitating dispersion of the particles.

A number of fluorinated ethers are presently being administered as anaesthetic agents via inhalation during surgical procedures. The relatively large quantities (up to 200 gms) which are used during surgical procedures indicates the low-toxicity of the group.

Additionally, their densities are ideally matched to the densities of glasses used in the formulations described above. For example, referring to the designations of 3M Limited:
HFE 7500 has a density of 1.61,
HFE 7200 has a density of 1.43, and
HFE 7100 has a density of 1.52.

These values are, co-incidentally similar to the density of sugar glass, which is about 1.5.

An additional benefit of using the invention is that fluorinated ethers, whilst being highly stable in normal conditions, are unstable when exposed to strong ultraviolet radiation such as is present in the stratosphere. This avoids a problem associated with perfluorocarbons which are known to contribute to the damaging "greenhouse" effect when released into the atmosphere after use.

Yet another advantage of the invention is that fluorinated ethers are relatively inexpensive and are readily available at a high degree of purity, greater than 98%. This compares with PFCs for which a typical example might have a purity of only about 55%.

Because fluorinated ethers are so well matched with the glasses, it has become possible to adopt a new approach to density matching. Previously, the glass was formulated, by use of additives, to match its density to that of the liquid PFC. However, it now becomes unnecessary to constrain the selection of the glass according to the need to achieve the correct density. The invention makes it possible to select the ideal glass/active ingredient composition; and then to mix a fluorinated ether possibly with the addition of small quantities of PFCs or other liquids so as to match the density of the liquid to the density of the particles. It even becomes practicable to take ready-made compositions of active ingredient preserved in a glassy substance; to grind it into particles and then to suspend it in a liquid matched to the density of the particles.

The densities of the particles and of the liquid do not have to be identical. However, they should be sufficiently close that Brownian movement or other thermodynamic influences keep the particles in suspension.

Because the particles have been found to disperse so effectively in fluorinated ethers and other liquids referred to above, the need to make the particles as small as possible, so as to maintain a suspension, is now not as acute as before. Specialist, modified spray drying techniques, which were previously thought by the inventors to be needed in order to achieve small particle size, are now unnecessary although the standard commercial spray drying process is still one possible technique for making the particles. However, alternative methods such as freeze drying or grinding would now also be practicable. It is only necessary that the particles should be sufficiently small to permit passage through a hypodermic syringe.

It is envisaged that the invention will normally be employed for the formulation of vaccines, therapeutic proteins or other medications for injection through the skin of a patient. However, other uses for the invention may be possible, eg for medicinal liquids which are administered orally or inhaled after atomising. It is also possible that there may be non-medicinal uses for the invention which is generally applicable to any situation where it is desired to preserve a biologically active material in a glassy solid and where there is a need for the composition to be presented in liquid form.

One way of performing the invention will now be described.

Sterile, bulk liquid hepatitis B vaccine with aluminium hydroxide adjuvant was obtained from Panacea Biotech of Delhi. This was mixed with sterile colloidal calcium phosphate suspension and raffinose solution in the correct proportions to give a single adult dose of 10 µg vaccine in 50 milligrams of total solids. The proportion of calcium phosphate to raffinose was calculated to give solid glass particles with a density matching that of the hydrofluoroether HFE 7,500 of 1.61 Kg/L. While being constantly stirred by a magnetic stirrer, this suspension was pumped through a two fluid nozzle at the rate of 2 ml per minute with a nozzle gas flow of 2.5 Kg/hr. The resulting droplets were dried in the chamber of a GEA Niro SD Micro spray with a heated air flow of 30 Kg per hour. The outlet temperature was maintained at 90°C by regulating the inlet temperature keeping the feed flow rate constant. Product was collected in a sterile bottle and transferred to a laminar flow hood with class 100 air flow. Sterile HFE 7,500 was added at the rate of 1 ml per 100 mg of powder and agitated in a frequency sweep ultrasonic bath for 10 min to fully disperse the microspheres. In the flow hood, the liquid was dispensed in 0.6 ml volumes into sterile 2 ml serum vials, plugged with neoprene stoppers and sealed with aluminium caps. The vaccine vials were used to set up a study of the in vitro stability of the vaccine at various storage temperatures.

## Claims

1. A formulation comprising an active ingredient preserved in glassy or amorphous particles, the particles being suspended in a liquid in which at least one component comprises a fluorinated ether.

2. A formulation according to Claim 1 in which the fluorinated ether is a hydrofluoroether or hydrofluoropolyether.

3. A formulation according to Claim 1 or 2 in which the particles contain a sugar glass or a glass which is a mixture of sugar, metal carboxylate, amino acid and or calcium phosphate or any combination of these.

4. A formulation according to any preceding claim in which the particles have a density which is matched to the density of the liquid sufficiently closely that the particles will remain in suspension under normal conditions.

5. A formulation according to any preceding claim in which the liquid contains different components specified in claim 1 mixed in proportions to give a required density.

6. A formulation according to any preceding claim in which the liquid contains a perfluorocarbon mixed with one or more components specified in claim 1.

7. A formulation according to any preceding claim in which the active ingredient is a vaccine.

8. A formulation according to any preceding claim in which the particles are made by spray drying.

9. A formulation according to any one of Claims 1 to 7 in which the particles are made by freeze drying.

10. A formulation according to any one of Claims 1 to 7 in which the particles are made by grinding.

11. A method of making a formulation according to Claim 5 or 6 including the step of selecting liquids to give the required density matching properties and mixing them with the particles.

12. A formulation comprising an active ingredient preserved in glassy or amorphous particles, the particles being suspended in a liquid comprising a hydrofluoroether.

## Patentansprüche

1. Formulierung, umfassend einen Wirkstoff, der in glasartigen oder amorphen Teilchen konserviert ist, wobei die Teilchen in einer Flüssigkeit suspendiert sind, in welcher wenigstens eine Komponente einen fluorierten Ether umfasst.

2. Formulierung nach Anspruch 1, in welcher der fluorierte Ether ein Hydrofluorether oder Hydrofluorpolyether ist.

3. Formulierung nach Anspruch 1 oder 2, in welcher die Teilchen ein Zuckerglas oder ein Glas, welches eine Mischung aus Zucker, Metallcarboxylat, Aminosäure und oder Calciumphosphat oder einer beliebigen Kombination von diesen ist, enthalten.

4. Formulierung nach einem der vorangehenden Ansprüche, in welcher die Teilchen eine Dichte aufweisen, welche an die Dichte der Flüssigkeit ausreichend nahe angeglichen ist, dass die Teilchen unter normalen Bedingungen in Suspension bleiben.

5. Formulierung nach einem der vorangehenden Ansprüche, in welcher die Flüssigkeit verschiedene Komponenten, die in Anspruch 1 angegeben sind, in solchen Verhältnissen vermischt enthält, dass sie eine erforderliche Dichte ergeben.

6. Formulierung nach einem der vorangehenden Ansprüche, in welcher die Flüssigkeit einen Perfluorkohlenstoff mit einer oder mehreren Komponenten, die in Anspruch 1 angegeben sind, vermischt enthält.

7. Formulierung nach einem der vorangehenden Ansprüche, in welcher der Wirkstoff ein Impfstoff ist.

8. Formulierung nach einem der vorangehenden Ansprüche, in welcher die Teilchen durch Sprühtrocknen hergestellt sind.

9. Formylierung nach einem der Ansprüche 1 bis 7, in welcher die Teilchen durch Gefriertrocknen hergestellt sind.

10. Formulierung nach einem der Ansprüche 1 bis 7, in welcher die Teilchen durch Mahlen hergestellt sind.

11. Verfahren zum Herstellen einer Formulierung nach Anspruch 5 oder 6, das den Schritt der Auswahl von Flüssigkeiten so, dass die erforderlichen Dichteangleichungseigenschaften erhalten werden, und des Vermischens dieser mit den Teilchen einschließt.

12. Formulierung, umfassend einen Wirkstoff, der in glasartigen oder amorphen Teilchen konserviert ist, wobei die Teilchen in einer Flüssigkeit suspendiert sind, die einen Hydrofluorether umfasst.

## Revendications

1. Formule comprenant un ingrédient actif conservé dans des particules vitreuses ou amorphes, les particules étant suspendues dans un liquide dans lequel au moins un composant comprend un éther fluoré.

2. Formule selon la revendication 1, dans laquelle l'éther fluoré est un hydrofluoroéther ou un hydrofluoropolyéther.

3. Formule selon la revendication 1 ou 2, dans laquelle les particules contiennent un verre de sucre ou un verre qui est un mélange de sucre, d'un carboxylate de métal, d'un acide aminé et/ou de phosphate de calcium, ou l'une quelconque de leurs combinaisons.

4. Formule selon l'une quelconque des revendications précédentes, dans laquelle les particules ont une densité qui est suffisamment proche de la densité du liquide pour que les particules restent en suspension dans des conditions normales.

5. Formule selon l'une quelconque des revendications précédentes, dans laquelle le liquide contient différents composants spécifiés dans la revendication 1 mélangés en des proportions permettant de donner une densité requise.

6. Formule selon l'une quelconque des revendications précédentes, dans laquelle le liquide contient un perfluorocarbone mélangé avec un ou plusieurs composants spécifiés dans la revendication 1.

7. Formule selon l'une quelconque des revendications précédentes, dans laquelle l'ingrédient actif est un vaccin.

8. Formule selon l'une quelconque des revendications précédentes, dans laquelle les particules sont préparées par séchage par pulvérisation.

9. Formule selon l'une quelconque des revendications 1 à 7, dans laquelle les particules sont préparées par lyophilisation.

10. Formule selon l'une quelconque des revendications 1 à 7, dans laquelle les particules sont préparées par broyage.

11. Procédé de préparation d'une formule selon la revendication 5 ou 6, comprenant l'étape consistant à choisir des liquides pour donner les propriétés requises permettant de faire correspondre les densités et à les mélanger avec les particules.

12. Formule comprenant un ingrédient actif conservé dans des particules vitreuses ou amorphes, les particules étant suspendues dans un liquide comprenant un hydrofluoroéther.
